# EUROPEAN PATENT APPLICATION

(11) **EP 4 461 240 A1**
(43) Date of publication of application: **13.11.2024**
(21) Application number: 23858713.3
(22) Date of filing: 04.05.2023
(51) Int. Cl.: A61B 17/29

(54) **LINE DRIVE MOTION MODULE AND MINIMALLY INVASIVE SURGICAL FORCEPS**

(30) Priority: 31.08.2022 CN 202211053319
(71) Applicant: Jingqin Zhizao (Suzhou) Medical Technology Co., Ltd., Suzhou, Jiangsu 215301 (CN)
(72) Inventor: XU, Xinliangyi, Suzhou, Jiangsu 215301 (CN); LI, Xiaozhen, Suzhou, Jiangsu 215301 (CN); ZHOU, Xiaojun, Suzhou, Jiangsu 215301 (CN); WANG, Yichu, Suzhou, Jiangsu 215301 (CN)
(74) Representative: J A Kemp LLP
(86) International application number: PCT/CN2023/092062
(87) International publication number: WO 2024/045673

(57) **Abstract**

Provided are a line-driven motion module and a pair of minimally invasive surgical forceps. The line-driven motion module comprises a first adapter structure (1) and a second adapter structure (2) which are spaced apart from each other, a positioning structure (3), a joint assembly, and control lines (6). The positioning structure (3) is arranged between the first adapter structure (1) and the second adapter structure (2). The joint assembly comprises a plurality of interconnected joint units (5). Adjacent joint units (5) are configured to be movably connected to each other. The joint assembly is mechanically coupled and connected between the positioning structure (3) and each of the first and second adapter structures. At least two control lines (6) are provided. The line-driven motion module of the structure can be switched between bent states and an initial state. The first adapter structure (1) and the second adapter structure (2) move correspondingly around the positioning structure (3), thereby achieving the motion action and position expected by the working conditions. The overall module structure thereof has the advantages of continuous transmission and stable motion. (FIG 2)

## Description

### CROSS-REFERENCE TO RELATED APPLICATIONS

The present application claims priority to Chinese Patent Application No. 202211053319.1, filed to the China Patent Office on August 31, 2022 and entitled "LINE-DRIVEN MOTION MODULE AND MINIMALLY INVASIVE SURGICAL FORCEPS", the entire content of which is incorporated herein by reference.

### TECHNICAL FIELD

The present application relates to the technical field of medical instruments, in particular to a line-driven motion module and minimally invasive surgical forceps.

### BACKGROUND

A joint structure is widely applied in the fields of machinery and medical care due to its good strength and capability of flexible adaptability to space motion. At present, in medical instruments, for example, in minimally invasive surgery, a doctor enables a minimally invasive apparatus to perform expected actions according to control actions and instructions through a corresponding operating mechanism, so as to achieve the purpose of medical treatment and diagnosis for a human body.

In the prior art, the minimally invasive apparatus is usually composed of a joint structure and a plurality of control lines, the control lines and the joint structure are connected inside, one ends of the plurality of control lines are connected with an external driving device, and the other ends of the plurality of control lines are connected with an external operating end. When a joint structure of a minimally invasive machine needs to be adjusted to bend, the control lines are withdrawn through the external driving device, adjacent joints where a line withdrawing end is located approach to one another, and an included angle between the adjacent joints is reduced; the control lines are pushed and extended through the external driving device, adjacent joints where a line pushing end is located move away from one another, and an included angle between the adjacent joints is increased; and accordingly, bending and folding actions of the joints are completed, and an expected operating process is achieved.

However, as for the above joint structure, cooperation of line drawing and line retracting is performed on the plurality of control lines through a driving end of a driving device at the same time, so as to make the joints bend; and the control lines laterally abut against the joints during line drawing and line retracting specifically, so a tiny run-out error may exist between the joints, after the error is accumulated and superposed, run-out of the whole joint structure may be formed, as a consequence, transmission stability of the joint structure is poor, and there is a problem of instability during performing operations.

### SUMMARY OF THE INVENTION

A technical problem to be solved by the present application is a defect that in the prior art, transmission stability of a joint structure is poor, and there is instability during performing operations.

In a first aspect, the present application provides a line-driven motion module, including:
a first adapter structure and a second adapter structure which are spaced apart from each other, wherein the first adapter structure is configured to be connected with an external driving mechanism, and the second adapter structure is configured to be connected with an operating mechanism;
a positioning structure, wherein the positioning structure is configured to be connected with an externally-connected support ring and arranged between the first adapter structure and the second adapter structure;
a joint assembly, wherein the joint assembly includes a plurality of interconnected joint units, adjacent joint units are configured to be movably connected to each other, and the joint assembly is mechanically coupled and connected between the positioning structure and each of the first and second adapter structures; and
at least two control lines, wherein two ends of each control line are fixedly connected with the first adapter structure and the second adapter structure, respectively, and the control lines are arranged in all of the joint units and the positioning structure in a penetrating manner to be movably connected with the joint units and the positioning structure;
the line-driven motion module has bent states in which the first adapter structure draws the control lines under an external force to enable the second adapter structure and the joint assembly to bend in a linked manner with respect to the first adapter structure, and an initial state in which the first adapter structure, the positioning structure and the second adapter structure are arranged coaxially; and the line-driven motion module is arranged to switch between the bent states and the initial state.

Optionally, in the line-driven motion module, the positioning structure includes:
a positioning body and at least two limiting channels formed in the positioning body, wherein the limiting channels are configured for allowing the control lines to penetrate therethrough.

Optionally, in the line-driven motion module,
an extending direction of each of the limiting channels is arranged parallel to an extending direction of the positioning body; and
the line-driven motion module has a first bent state in which the first adapter structure draws the control lines under an external force to enable the second adapter structure and the joint assembly to bend in a linked manner to move in an S shape synchronously with respect to the first adapter structure.

Optionally, in the line-driven motion module, in the first bent state, a first end surface, remote from a side thereof connected to the joint units, of a first adapter body, is arranged parallel to a second end surface, remote from a side thereof connected to the joint units, of a second adapter body.

Optionally, in the line-driven motion module, each of the limiting channels has a first lead port at a side close to the first adapter structure and a second lead port at a side close to the second adapter structure, and a first plane containing the first lead port and an axis of the positioning structure is oriented at an angle of 90 degrees to a second plane containing the second lead port and the axis of the positioning structure; and
the line-driven motion module has a second bent state in which the first adapter structure draws the control lines under an external force to enable the second adapter structure and the joint assembly to bend in a linked manner to move in a noncoplanar S shape synchronously with respect to the first adapter structure.

Optionally, in the line-driven motion module, each of the limiting channels has a third lead port at a side close to the first adapter structure and a fourth lead port at a side close to the second adapter structure, and a third plane containing the third lead port and an axis of the positioning structure is oriented at an angle of 180 degrees to a fourth plane containing the fourth lead port and the axis of the positioning structure; and
the line-driven motion module has a third bent state in which the first adapter structure draws the control lines under an external force to enable the second adapter structure and the joint assembly to bend in a linked manner to move in a C shape synchronously with respect to the first adapter structure.

Optionally, in the line-driven motion module, each limiting channel includes a first straight segment, a second straight segment and a curved segment; the first straight segment is arranged on a side of the positioning body close to the first adapter structure, and the second straight segment is arranged on another side of the positioning body close to the second adapter structure; an extending direction of the first straight segment and an extending direction of the second straight segment are both arranged parallel to an extending direction of the positioning body; and the curved segment is arranged between and in communication with the first straight segment and the second straight segment.

Optionally, in the line-driven motion module, the positioning structure further includes a first connecting hole extending in the same direction as an extending direction of the positioning structure;
each of the adapter structures has a second connecting hole extending in the same direction as an extending direction of the corresponding adapter structure; and
each of the joint units is provided with a core hole, and all the core holes, all the second connecting holes and all the first connecting holes together form an intervention channel configured to allow an externally-connected pipe to penetrate therethrough.

Optionally, the line-driven motion module further includes a swiveling state in which the first adapter structure is rotated under an external force to draw the control lines and enable the second adapter structure and the joint assembly to twist synchronously with the first adapter structure.

Optionally, in the line-driven motion module, the positioning structure further includes:
a first joining portion arranged at an end, close to the first adapter structure, of the positioning body, wherein, the first joining portion is movably connected to the joint units; and
a second joining portion arranged at another end, close to the second adapter structure, of the positioning body, wherein, the second joining portion is configured to be movably connected to the joint units.

Optionally, in the line-driven motion module, the first adapter structure includes a third joining portion, wherein, the third joining portion is movably connected to the joint units; and
the second adapter structure includes a fourth joining portion, wherein, the fourth joining portion is movably connected to the joint units.

Optionally, in the line-driven motion module, each of the adapter structures further includes a fitting hole and a fixing portion, wherein a corresponding control line is arranged in the fitting hole in a penetrating manner, and the fixing portion adjoins the fitting hole, and the fixing portion is fixedly connected to an end of the corresponding control line.

Optionally, the line-driven motion module includes at least four control lines, and all the control lines are distributed to be rotationally symmetrical with respect to an axis of the positioning structure.

Optionally, in the line-driven motion module, each joint unit includes:
a rotating member, provided with limiting portions in a circumferential direction; and
a limiting base fixedly connected to the rotating member, wherein the limiting base is internally provided with a receiving cavity for being movably connected to the rotating member of an adjacent joint unit, the receiving cavity is further internally provided with limiting members, and the limiting members cooperate with the limiting portions of the adjacent joint unit to limit axial rotation of the rotating member of the adjacent joint unit.

Optionally, in the line-driven motion module, the rotating member is a sphere, and an inner wall surface of the receiving cavity is fitted with an outer wall surface of the rotating member of the adjacent joint unit;
Optionally, in the line-driven motion module, two limiting portions are provided, the two limiting portions are spaced apart from each other so as to form a fitting space for limiting the limiting members, and the limiting members are movably connected into the fitting space.

Optionally, in the line-driven motion module, each of the limiting members is a column, wherein, an extending direction of the column is arranged to perpendicularly intersect a plane containing a planar surface of each of the limiting portions.

Optionally, in the line-driven motion module, each of the joining portions has a structure identical to that of the rotating member or the limiting base.

In a second aspect, the present application provides minimally invasive surgical forceps, including a support ring and the line-driven motion module in the first aspect.

The technical solutions provided by the present application have the following advantages.
1. The line-driven motion module provided by the present application includes the first adapter structure and the second adapter structure which are spaced apart from each other, the positioning structure, the joint assembly and the control lines, the first adapter structure is configured to be connected with the external driving mechanism, and the second adapter structure is configured to be connected with the operating mechanism; the positioning structure is configured to be connected with the externally-connected support ring and arranged between the first adapter structure and the second adapter structure; the joint assembly includes the plurality of interconnected joint units, the adjacent joint units are configured to be movably connected to each other, and the joint assembly is mechanically coupled and connected between the positioning structure and each of the first and second adapter structures; at least two control lines are provided, two ends of each control line are fixedly connected with the first adapter structure and the second adapter structure, respectively, and the control lines are arranged in all of the joint units and the positioning structure in a penetrating manner to be movably connected with the joint units and the positioning structure; the line-driven motion module has the bent states in which the first adapter structure draws the control lines under an external force to enable the second adapter structure and the joint assembly to bend in a linked manner with respect to the first adapter structure, and the initial state in which the first adapter structure, the positioning structure and the second adapter structure are arranged coaxially; and the line-driven motion module is arranged to switch between the bent states and the initial state.
   In the line-driven motion module of this structure, the external driving mechanism adjusts motion of the first adapter structure, so that the control lines and the joint assembly cooperate to drive the second adapter structure, the joint assembly is composed of the plurality of interconnected joint units, the adjacent joint units are configured to be movably connected to each other, and the control lines are drawn by motion of the first adapter structure to correspondingly adjust the adjacent joint units so as to adapt to motion of the first adapter structure; motion of the control lines are positioned and guided through the positioning structure, and the joint assembly and the adapter structures connected to two ends of the positioning structure bend and transform by using the positioning structure as a reference; when the line-driven motion module is switched between the bent states and the initial state, the first adapter structure and the second adapter structure move correspondingly around the positioning structure, an action force and a torque are transferred through connection of the control lines, so that the second adapter structure and the first adapter structure can be induced to be in linkage, and the second adapter structure achieves expected motion actions and positions; and through whole module design, stability of the control lines driving the joint units is improved, motion is continuous, and the tiny run-out error between the adjacent joints can be avoided.
2. In the line-driven motion module provided by the present application, the positioning structure includes the positioning body and at least two limiting channels formed in the positioning body, wherein the limiting channels are configured for allowing the control lines to penetrate therethrough.
   In the line-driven motion module of this structure, the limiting channels are configured for allowing the control lines to penetrate therethrough, motion of the control lines is limited and guided through the limiting channels, a structural design is made for the limiting channels, so that when the control lines are drawn by the first adapter structure, stress conditions of the second adapter structure and the joint assembly connected between the positioning structure and the second adapter structure is changed, the joint assembly and the adapter structures connected to the two ends of the positioning structure bend and transform by using the positioning structure as a reference, thus the joint assembly achieves expected actions and positions, so as to adapt to an expected working condition.
3. In the line-driven motion module provided by the present application, the positioning structure further includes a first connecting hole extending in the same direction as an extending direction of the positioning structure, and each of the adapter structures has a second connecting hole extending in the same direction as an extending direction of the corresponding adapter structure; and each of the joint units is provided with a core hole, and all the core holes, all the second connecting holes and all the first connecting holes together form an intervention channel configured to allow an externally-connected pipe to penetrate therethrough.
   In the line-driven motion module of this structure, fitting holes are configured in the adapter structures, the joint structure and the positioning structure, so as to form an intervention channel for the purpose of medical treatment, a flexible pipe may be connected into the intervention channel through an operation space formed by the intervention channel, and when the line-driven motion module has a bending change, the pipe also bends, so as to meet the expected working condition.
4. In the line-driven motion module provided by the present application, the positioning structure further includes a first joining portion and a second joining portion, the first joining portion is arranged at an end, close to the first adapter structure, of the positioning body, and the first joining portion is movably connected to the joint units; and the second joining portion is arranged at another end, close to the second adapter structure, of the positioning body, and the second joining portion is configured to be movably connected to the joint units.

In the line-driven motion module of this structure, when the line-driven motion module needs to be in the bent states, the support ring is fixedly connected with the positioning body, the adjacent joint unit is limited through the first joining portion and the second joining portion, and it is guaranteed that cooperation action with the adjacent joint unit is stable, so as to induce the joint assembly to bend; when the line-driven motion module needs to be in a swiveling state, the first adapter structure draws the control lines to co-rotate with the adjacent joint assembly, and the first joining portion receives transmission of the adjacent joint unit, so as to cause the positioning structure to rotate synchronously; and the joint unit between the second adapter structure and the positioning structure and the second adapter structure are driven through the second joining portion, so as to cause the whole line-driven motion module to self-rotate.

### BRIEF DESCRIPTION OF THE DRAWINGS

In order to more clearly describe specific implementations of the present application or technical solutions in the prior art, the accompanying drawings needed by the description in the specific implementations or in the prior art will be briefly introduced below. Apparently, the accompanying drawings in the following description are some implementations of the present application. Those ordinarily skilled in the art can also obtain other accompanying drawings according to these accompanying drawings without making creative work.
FIG 1 is a schematic diagram of connection of a line-driven motion module and a support ring provided by an embodiment of the present application.
FIG 2 is a schematic structural diagram of a line-driven motion module provided by an embodiment of the present application.
FIG 3 is a schematic structural diagram of a first adapter structure in a line-driven motion module provided by an embodiment of the present application.
FIG 4 is a schematic structural side view of a first adapter structure in a line-driven motion module provided by an embodiment of the present application.
FIG 5 is a schematic structural top view of a first adapter structure in a line-driven motion module provided by an embodiment of the present application.
FIG 6 is a schematic structural bottom view of a first adapter structure in a line-driven motion module provided by an embodiment of the present application.
FIG 7 is a schematic three-dimensional structural diagram of a positioning structure in a line-driven motion module provided by an embodiment of the present application.
FIG 8 is a schematic structural side view of a positioning structure in a line-driven motion module provided by an embodiment of the present application.
FIG 9 is a schematic structural front view of a positioning structure in a line-driven motion module provided by an embodiment of the present application.
FIG 10 is a schematic structural diagram of a put-through channel serving as a limiting channel in a positioning structure in a line-driven motion module provided by an embodiment of the present application.
FIG 11 is a schematic structural diagram of a line-driven motion module in a first bent state provided by an embodiment of the present application.
FIG 12 is a schematic structural top view of a line-driven motion module in a first bent state provided by an embodiment of the present application.
FIG 13 is a schematic structural diagram of a joint unit in a line-driven motion module provided by an embodiment of the present application.
FIG 14 is a schematic structural front view of a joint unit in a line-driven motion module provided by an embodiment of the present application.
FIG 15 is a schematic three-dimensional diagram of a joint unit in a line-driven motion module provided by an embodiment of the present application.
FIG 16 is a three-dimensional diagram of a joint unit in a line-driven motion module provided by an embodiment of the present application.
FIG 17 is a schematic structural diagram of a positioning structure when a line-driven motion module provided by an embodiment of the present application is in a first bent state.
FIG 18 is a schematic structural side view of a positioning structure when a line-driven motion module provided by an embodiment of the present application is in a first bent state.
FIG 19 is a schematic structural diagram of a line-driven motion module in a second bent state provided by an embodiment of the present application.
FIG 20 is a schematic structural top view of a line-driven motion module in a second bent state provided by an embodiment of the present application.
FIG 21 is a schematic structural diagram of a positioning structure when a line-driven motion module provided by an embodiment of the present application is in a third bent state.
FIG 22 is a schematic structural side view of a positioning structure when a line-driven motion module provided by an embodiment of the present application is in a third bent state.

### Descriptions of reference numerals:

1-first adapter structure; 11-first adapter body; 111-first end surface; 112-second connecting hole; 113-fitting hole; 114-fixing portion; 12-third joining portion;
2-second adapter structure; 21-second adapter body; 211-second end surface; 22-fourth joining portion;
3-positioning structure; 31-positioning body; 311-first connecting hole; 32-limiting channel; 3201-first lead port; 3202-second lead port; 3203-third lead port; 3204-fourth lead port; 321-first straight segment; 322-second straight segment; 323-curved segment; 33-first joining portion; 34-second joining portion;
4-support ring;
5 joint unit; 51-rotating member; 511-limiting portion; 52-limiting base; 521-receiving cavity; 522-core hole; 523-threading hole; 53-limiting member; and 6-control line.

### DETAILED DESCRIPTION

The technical solutions of the present application are clearly and completely described in the following with reference to the accompanying drawings. Apparently, the described embodiments are merely some rather than all of the embodiments of the present application. All other embodiments obtained by those ordinarily skilled in the art based on the embodiments of the present application without making creative efforts fall within the protection scope of the present application.

In the description of the present application, it needs to be noted that directions or position relations indicated by terms such as "center", "upper", "lower", "left", "right", "vertical", "horizontal", "inner" and "outer" are directions or position relations as shown in the accompanying drawings and are only intended to conveniently describe the present application and simplify the description but not to indicate or imply that an apparatus or element referred to necessarily has a specific direction or is constructed or operated in a specific direction, so as not to be understood as a limitation on the present application. Besides, terms such as "first", "second" and "third" are only used for the description instead of being understood as indicating or implying a relative significance.

In the description of the present application, it needs to be noted that unless otherwise specified and defined clearly, terms such as "connection", "connected", "connecting" are to be understood in a broad sense, for example, it may be a fixed connection, a detachable connection, or an integrated connection; it may be a mechanical connection or an electrical connection; and it may be a direct connection, or an indirect connection through an intermediate medium, or internal communication between two elements. Specific meanings of the above terms in the present application may be understood by those ordinarily skilled in the art according to specific conditions.

In the following description, when in use, a side of an apparatus close to an operator is a proximal end, and an end away from the operator is a distal end.

Besides, technical features involved in different implementations of the present application described below may be combined mutually without a conflict between each other.

### Embodiment 1

This embodiment provides a line-driven motion module, which can be configured for a transmission structure of a medical instrument and serve as a mechanical member for the purpose of medical treatment and diagnosis, for example, a minimally invasive surgical instrument is operated in an narrow space to adapt to motion forms during surgery, so as to meet a use working condition of a medical environment. The line-driven motion module may be configured for constituting a transmission mechanism, is applied in a scene that needs to transfer rotation motion with a variable axis and has a demand for antitorque and strength, and certainly may also be suitable for an application environment such as a human body joint, a robot and a robot joint so as to conveniently build and fabricate the robot and the robot joint.

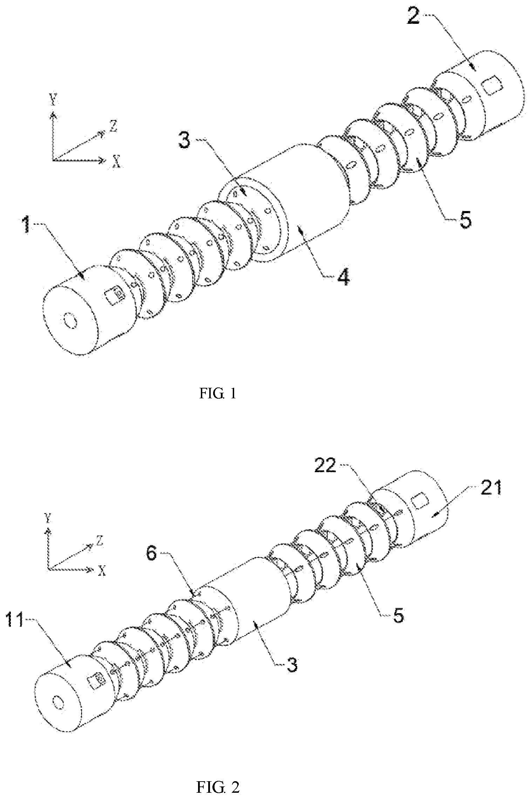

As shown in FIG 1 and FIG 2, the line-driven motion module includes a first adapter structure 1 and a second adapter structure 2, a positioning structure 3, a joint assembly and control lines 6.

The first adapter structure 1 is configured to be connected with an external driving mechanism. The external driving mechanism adjusts motion of the first adapter structure 1, so that the first adapter structure 1 may move along an X-Y plane in FIG 1 and FIG 2 and rotate around a Z axis to move in a three-dimensional space provided by the outside.

The second adapter structure 2 is configured to be connected with an operating mechanism. When the first adapter structure 1 moves, the control lines 6 cooperate with the joint assembly to drive the second adapter structure 2, so that the operating mechanism and the second adapter structure 2 adapt to actions and positions when the driving mechanism adjusts motion of the first adapter structure 1.

The line-driven motion module provided by this embodiment has bent states in which the first adapter structure 1 draws the control lines 6 under action of the driving mechanism to enable the second adapter structure 2 and the joint assembly to bend in a linked manner with respect to the first adapter structure 1. The line-driven motion module further has an initial state in which the first adapter structure 1 draws the control lines 6 under action of the driving mechanism to enable the first adapter structure 1, the positioning structure 3 and the second adapter structure 2 to be arranged coaxially. The line-driven motion module is arranged to switch between the bent states and the initial state.

The line-driven motion module provided by the present application, as shown in FIG 1, further includes a swiveling state in which the first adapter structure 1 is rotated under driving action of the driving mechanism to draw the control lines 6 and enable the second adapter structure 2 and the joint assembly to twist synchronously with the first adapter structure 1. The support ring 4 is rotationally connected with the positioning body 31.

The first adapter structure 1 and the second adapter structure 2 may be replaced equivalently to be connected with the driving mechanism, during specific fitting and use, one of the first adapter structure and the second adapter structure may be connected with the external driving mechanism, the other one may be connected with the operating mechanism, and convenience of connection of a module structure may be improved.

As shown in FIG 1 to FIG 6, the first adapter structure 1 includes a first adapter body 11, the second adapter structure 2 includes a second adapter body 21, and the first adapter body 11 and the second adapter body 21 are spaced apart from each other; and the first adapter body 11 has a first end surface 111 remote from a side thereof connected to the joint units 5, the second adapter body 21 has a second end surface 211 remote from a side thereof connected to the joint units 5. The first end surface 111 is located on a proximal end portion of the line-driven motion module. The second end surface 211 is located on a distal end portion of the line-driven motion module, and in the initial state of the line-driven motion module, the first end surface 111 is arranged opposite to the second end surface 211.

It needs to be noted that when the line-driven motion module is driven to move specifically, the first end surface 111 may be used as a reference surface of driving of the driving mechanism, the driving mechanism drives the first adapter structure 1 for position adjustment, the whole line-driven motion module moves in a three-dimensional space to implement a corresponding bending action pose, then the first end surface 111 is adjusted through the driving mechanism so that the first end surface 111 and the second end surface 211 perform corresponding actions, a purpose of adjusting the position and orientation of the second end surface 211 is achieved, and thus the operating mechanism on the second adapter structure 2 can perform actions of a pre-determined target position and target direction. Correspondingly, the first end surface 111 is adjusted through the driving mechanism so that the second end surface 211 performs actions corresponding to the first end surface 111, then the driving mechanism drives the first adapter structure 1 for position adjustment, and thus the whole line-driven motion module moves in the three-dimensional space to implement the corresponding bending action posture. The first end surface 111 may be used as a mounting surface for being connected with the driving mechanism, and a shape of the first end surface 111 may be a plane, a curved surface or a stepped surface, which is not limited specifically here.

In this embodiment, the first adapter body 11 and the second adapter body 21 are of rotary body structures.

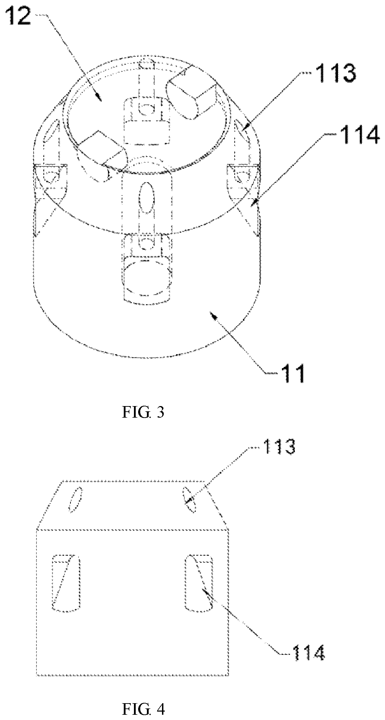

As shown in FIG 3, the first adapter structure 1 includes a third joining portion 12, and the third joining portion 12 is movably connected to the joint units 5. The third joining portion 12 is arranged at a distal end of the first adapter body 11, and an adjacent joint unit 5 at a distal end of the first adapter structure 1 is driven through the third joining portion 12.

As shown in FIG 2, the first adapter structure 2 includes a fourth joining portion 22, and the fourth joining portion 22 is movably connected to the joint units 5. The fourth joining portion 22 is arranged at a proximal end of the second adapter body 21, and a bending moment and a torque transmitted by the adjacent joint unit 5 at the proximal end of the second adapter structure 2 are connected and received through the fourth joining portion 22.

As shown in FIG 1, the positioning structure 3 is configured to be connected with an externally-connected support ring 4 and arranged between the first adapter structure 1 and the second adapter structure 2.

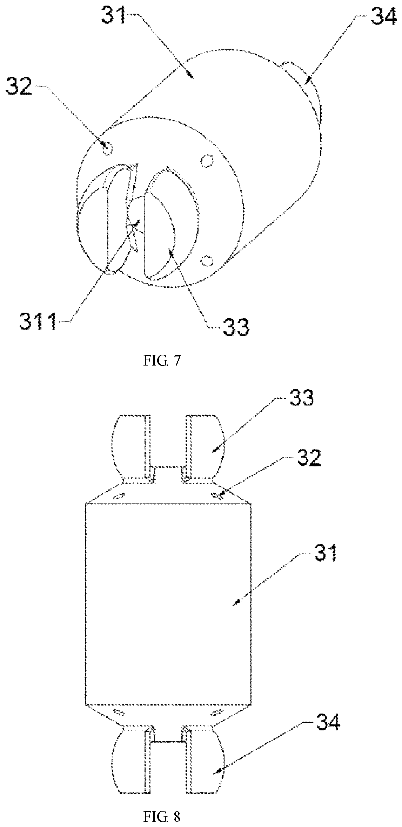

As shown in FIG 7, the positioning structure 3 includes a positioning body 31, and the positioning body 31 may be used as a supporting space for the control lines 6, so as to induce two ends of each control line 6 to bend and change according to motion of the first adapter structure 1.

As shown in FIG 7 to FIG 9, the positioning structure 3 includes the first joining portion 33 and the second joining portion 34, the first joining portion 33 is arranged at an end, close to the first adapter structure 1, of the positioning body 31, and the first joining portion 33 is movably connected to the joint units 5; and the second joining portion 34 is arranged at another end, close to the second adapter structure 2, of the positioning body 31, and the second joining portion 34 is configured to be movably connected to the joint units 5.

When the line-driven motion module needs to be in the bent states, the support ring 4 is fixed to the positioning body 31, and the adjacent joint unit 5 is limited through the first joining portion 33 and the second joining portion 34, so that it is guaranteed that cooperation action between the positioning body 31 and the adjacent joint unit is stable, and the joint assembly is induced to bend reliably.

When the line-driven motion module needs to be in the swiveling state, the first adapter structure 1 draws the control lines 6 and the adjacent joint assembly to co-rotate, the first joining portion 33 receives transmission from the adjacent joint unit 5, so as to cause the positioning structure 3 to rotate synchronously, and the joint unit 5 between the second adapter structure 2 and the positioning structure 3 and the second adapter structure 2 are driven through the second joining portion 34, so that the whole line-driven motion module is caused to self rotate.

In the line-driven motion module provided by the present application, as shown in FIG 1 and FIG 2, the joint assembly includes a plurality of interconnected joint units 5, adjacent joint units 5 are configured to be movably connected to each other, and the joint assembly is mechanically coupled and connected between the positioning structure 3 and each of the first and second adapter structures.

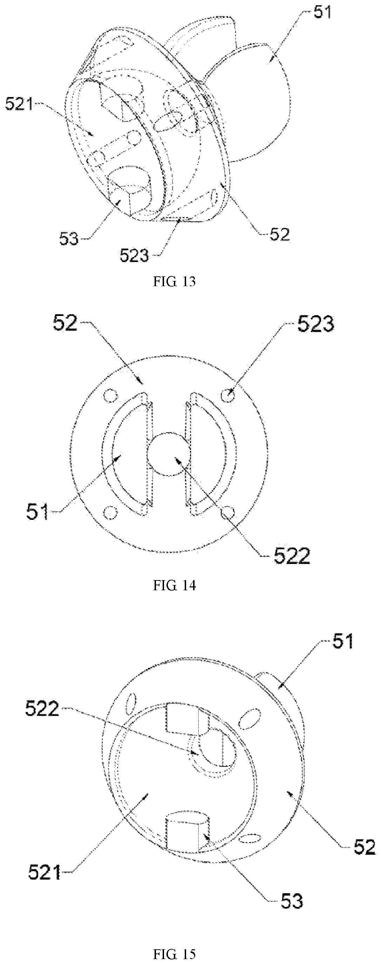

In this embodiment, as shown in FIG 13 to FIG 16, each joint unit 5 includes a rotating member 51 and a limiting base 52, and the rotating member 51 is provided with limiting portions 511 in a circumferential direction; and the limiting base 52 is fixedly connected to the rotating member 51, the limiting base 52 is internally provided with a receiving cavity 521 for being movably connected to the rotating member 51 of an adjacent joint unit 5, the receiving cavity 521 is internally provided with limiting members 53, and the limiting members 53 cooperate with the limiting portions 511 of the adjacent joint unit 5 to limit axial rotation of the rotating member 51 of the adjacent joint unit 5. The limiting members 53 and the limiting base 52 may be formed integrally and cooperate to form a space where the receiving cavity 521 is located. Each rotating member 51 is movably fitted with the corresponding receiving cavity 521, the rotating member 51 is arranged to be movably connected between the corresponding limiting member 53 and the corresponding receiving cavity 521, and the rotating member 51 is limited jointly by the corresponding limiting member 53 and the corresponding receiving cavity 521, so that the adjacent joint unit 5 is caused to bend in a circumferential direction.

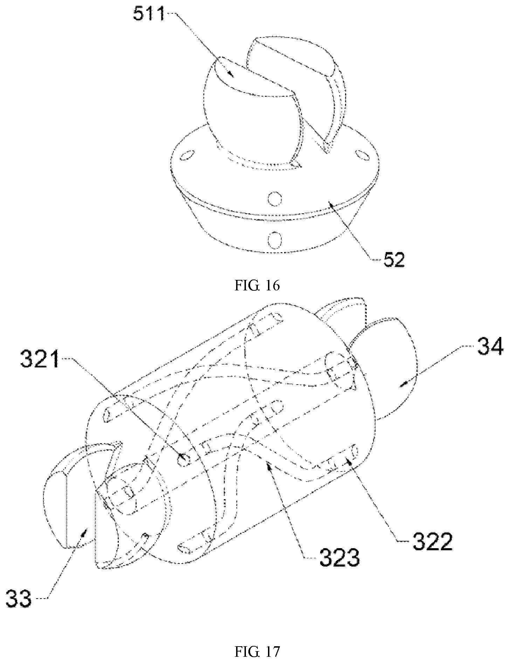

In this embodiment, as shown in FIG 16, two limiting portions 511 are provided, the two limiting portions 511 are spaced apart from each other so as to form a fitting space for limiting the limiting members 53, the limiting members 53 are movably connected into the fitting space, and the limiting members 53 rotationally abut against between the limiting portions 511.

In this embodiment, as shown in FIG 13 and FIG 16, each rotating member 51 has a spherical surface which may be formed through outer side wall surfaces of two limiting portions 511.

In this embodiment, as shown in FIG 13 and FIG 15, the receiving cavity 521 is a segment cavity, a wall surface of the segment cavity is fitted with and rotationally connected with an outer wall surface of the rotating member 51 of the adjacent joint unit 5, the receiving cavity 521 limits motion of the rotating member 51, and the rotating member 51 is caused to rotate with respect to an axial direction of the limiting member 53.

In the line-driven motion module provided by this embodiment, as shown in FIG 13 and FIG 15, each of the limiting members 53 is a column, an extending direction of the column is arranged to perpendicularly intersect a plane containing a planar surface of each of the limiting portions 511, the adjacent joint unit 5 is arranged orthogonally and has two perpendicular pivots, one of the pivots is parallel to the extending direction of the column, and the other one of the pivots is parallel to an intersection line between a radial plane of the column and the extending direction of the column.

In this embodiment, as shown in FIG 13 and FIG 14, each joint unit 5 is provided with threading holes 523 which are movably connected to the corresponding control line 6, and the control line 6 abuts against the threading holes 523 so as to transmit the adjacent joint unit 5.

In the line-driven motion module provided by this embodiment, in the positioning structure 3, the first joining portion 33 and the second joining portion 34 each have a structure identical to that of the rotating member 51, and in the first adapter structure 1 and the second adapter structure 2, the third joining portion 12 and the fourth joining portion 22 each have a structure identical to that of the limiting base 52. The line-driven motion module is good in transmission adaptability.

As an alternative implementation of this embodiment, in the positioning structure 3, the first joining portion 33 and the second joining portion 34 each have a structure identical to that of the limiting base 52, and in the first adapter structure 1 and the second adapter structure 2, the third joining portion 12 and the fourth joining portion 22 each have a structure identical to that of the rotating member 51. A structure of each joining portion is adaptively selected according to a connection manner of the joint assembly and actual transmission.

In the line-driven motion module provided by this embodiment, a joint unit 5 connected between the first adapter structure 1 and the positioning structure 3 is named a first joint assembly, and a joint unit 5 connected between the second adapter structure 2 and the positioning structure 3 is named a second joint assembly. The number of joint units 5 included in the first joint assembly and the number of joint units 5 included in the second joint assembly are not limited specifically.

In this embodiment, when the number of joint units 5 in the first joint assembly is greater than the number of joint units 5 in the second joint assembly, the line-driven motion module of this structure is good in flexibility, adjacent joint units 5 in the first joint assembly need to rotate for a smaller angle, and during a self-rotation transmission process, the smaller the angle for which the adjacent joint units 5 rotate is, transmission of self-rotation is better facilitated. In this case, while the first end surface 111 and the second end surface 211 remain a position relationship of being arranged originally in parallel in space or being arranged to extend and intersect at an included angle, the driving mechanism acts on the first adapter structure 1, so that the first joint assembly performs bending and stretching motion to adjust a pose of the line-driven motion module, and in this case, the second joint assembly and the second adapter structure 2 remain an original pose without a change.

In this embodiment, when the number of joint units 5 in the first joint assembly is the same as the number of joint units 5 in the second joint assembly, in the line-driven motion module of this structure, transmission between the first joint assembly and the second joint assembly is continuous, motion is consistent and synchronous, and it is easy to use and operate.

In this embodiment, when the number of joint units 5 in the first joint assembly is less than the number of joint units 5 in the second joint assembly, in the line-driven motion module of this structure, connection between the adjacent joint units 5 in the first joint assembly is tight, a structure is compact, and the connection has good rigidity, which is conducive to improving connection strength.

In the line-driven motion module provided by this embodiment, two or more control lines 6 are arranged, and all the control lines 6 are distributed to be rotationally symmetrical with respect to an axis of the positioning structure 3.

In this embodiment, the control lines 6 may be selected as an elastic fiber rope, a nickel-titanium wire and other elastic materials, so that the control lines 6 drive the joint assembly and the second adapter structure 2, and thus a linkage function of the line-driven motion module is achieved. In the initial state, when the joint units 5 at the two ends of the positioning structure 3 need to synchronously bend, all the control lines 6 are arranged in the same length, which is conducive to improving corresponding consistency of transmission, linkage and bending of the line-driven motion module.

In this embodiment, as shown in FIG 1, taking four control lines 6 as an example, the four control lines 6 are distributed on the line-driven motion module in a penetrating manner, respectively, and elastic coefficients of the four control lines 6 are consistent. In this embodiment, the two ends of each control line 6 are fixedly connected with the first adapter structure 1 and the second adapter structure 2 respectively, and the control lines 6 are arranged on all the joint units 5 and the positioning structure 3 in a penetrating manner to be movably connected with the joint units and the positioning structure 3.

In the line-driven motion module provided by this embodiment, the control lines 6 are drawn by motion of the first adapter structure 1 to correspondingly rotate and adjust the adjacent joint unit 5 and the second adapter body 21, so as to be adapted to rotation of the first adapter structure 1. The line-driven motion module abuts against the positioning body 31 through a middle segment of the corresponding control line 6, and when the first adapter structure 1 draws one of the corresponding control lines 6 to move, the second adapter structure 2 where the other end of the corresponding control line 6 is located is caused to perform fitting actions, so as to implement a linkage bending process.

In the line-driven motion module provided by this embodiment, the first adapter structure 1 and the second adapter structure 2 are the same, taking the first adapter structure 1 as an example, as shown in FIG 3 and FIG 4, the first adapter structure 1 further includes fitting holes 113 and fixing portions 114, the control lines 6 are arranged in the fitting holes 113 in a penetrating manner, the fixing portions 114 adjoin the fitting holes 113, the fixing portions 114 are fixedly connected with one ends of the control lines 6, and connection between the fixing portions 114 and the control lines 6 includes but is not limited to welding, bonding and locking through a fastening connector.

In the line-driven motion module provided by this embodiment, as shown in FIG 7 to FIG 9, the positioning structure 3 further includes limiting channels 32 formed in the positioning body 31, and the limiting channels 32 are configured for allowing the control lines 6 to penetrate therethrough. Two or more limiting channels 32 are provided, the number of the limiting channels 32 is the same as the number of control lines 6, and motion of the control lines 6 is limited and guided through the limiting channels 32.

In the line-driven motion module provided by this embodiment, a length and a material of the positioning body 31 are not limited specifically, the material of the positioning body 31 may be a steel tube, a medical plastic tube and the like, and a length and a material may be adaptively selected according to actual application occasions.

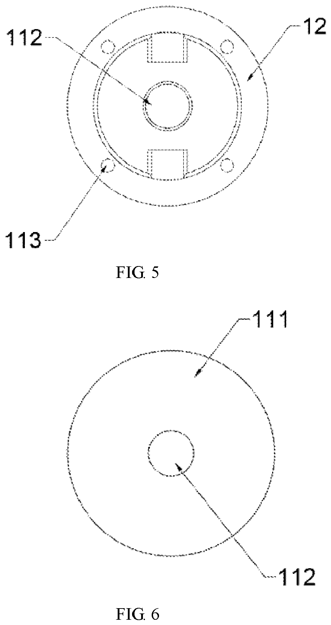

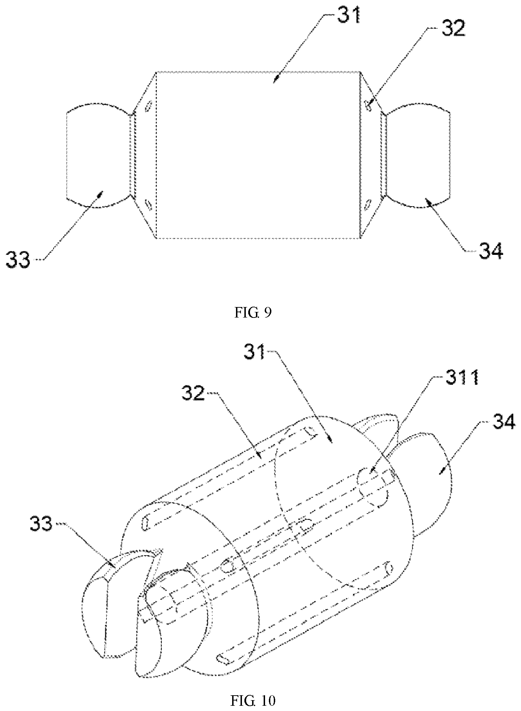

In the line-driven motion module provided by the present application, as shown in FIG 5 and FIG 6, each of the adapter structures has a second connecting hole 112 extending in the same direction as an extending direction of the corresponding adapter structure. As shown in FIG 7 and FIG 10, the positioning structure 3 further includes a first connecting hole 311 extending in the same direction as an extending direction of the positioning structure 3. As shown in FIG 14 and FIG 15, each of the joint units 5 is provided with a core hole 522, and all the core holes 522, all the second connecting holes 112 and all the first connecting holes 311 together form an intervention channel configured to allow an externally-connected pipe to penetrate therethrough. Fitting holes are configured in the adapter structures, the joint assembly and the positioning structure 3, which is intended to form an intervention channel for the purpose of medical treatment, a flexible pipe may be connected into the intervention channel through an operation space formed by the intervention channel, and when the line-driven motion module has a bending change, the pipe also bends, so as to meet the expected working condition.

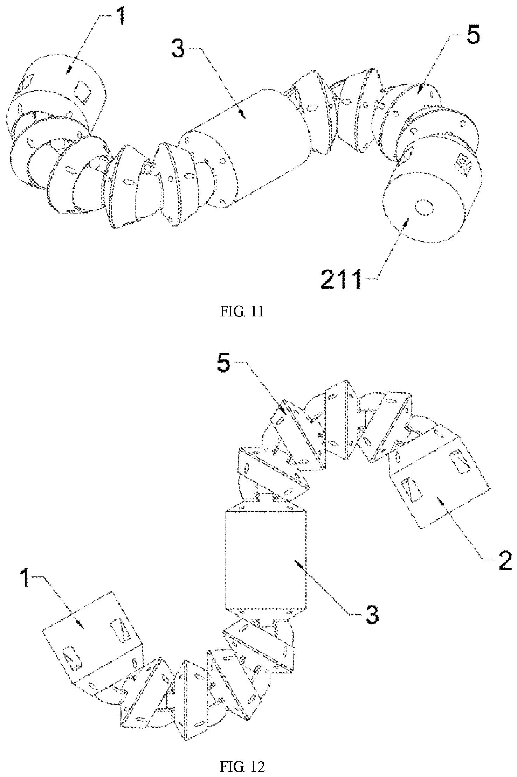

In the line-driven motion module provided by this embodiment, as shown in FIG 10, an extending direction of each of the limiting channels 32 is arranged parallel to an extending direction of the positioning body 31. The limiting channel 32 is a put-through channel. As shown in FIG 11 and FIG 12, the line-driven motion module has the first bent state in which the first adapter structure 1 draws the control lines 6 under driving action of the driving mechanism to enable the second adapter structure 2 and the joint assembly to bend in a linked manner to move in an S shape synchronously with respect to the first adapter structure 1. The first end surface 111 and the second end surface 211 remain an arrangement in parallel all the time, and by operating a position of the first end surface 111, the second end surface 211 is caused to perform the corresponding actions.

As shown in FIG 11 and FIG 12, in the first bent state, the first adapter structure 1 and the second adapter structure 2 move towards each other in opposite directions in an X-Y plane, and the first adapter structure 1 and the second adapter structure 2 relatively bend reversely, so that the joint assembly can achieve expected action poses and positions during an S-shape bending action process, to meet demanded working condition.

In the line-driven motion module provided by the present application, in the line-driven motion module, the first adapter structure 1 and the first joint assembly may bend with respect to the second adapter structure 2 and the second joint assembly, displacement generated by the driving mechanism acting on the first adapter structure 1 in a three-dimensional space with respect to the positioning structure 3 is implemented by compensation through mutual rotation of the adjacent connected joint units 5 between the first adapter structure 1 and the positioning structure 3 and self elastic extending and withdrawing of the control lines 6, so that the first joint assembly performs flexible bending and stretching actions; and in this case, the first end surface 111 and the second end surface 211 remain an original position relationship in parallel in space, and the second adapter structure 2 and the second joint assembly remain an original pose without a change. In a flexible motion range of the first joint assembly, the driving mechanism may drive the first adapter structure 1, so that the line-driven motion module performs bending and stretching adjustment on the first joint assembly in a position of an S-shape bending action process of the line-driven motion module, thus the first joint assembly may achieve the expected motion pose and the position during the bending and stretching action process, so as to meet the demanded working condition. Correspondingly, the driving mechanism may be firstly caused to drive the first adapter structure 1 so as to perform bending and stretching adjustment on the first joint assembly, then the driving mechanism is caused to drive the first adapter structure 1 so as to cause the whole line-driven motion module to generate an S-shape bending action, and thus the demanded working condition is met.

As an alternative implementation of this embodiment, each of the first joining portion 33 and the second joining portion 34 on the positioning structure 3 may be formed by the joint unit 5, so as to achieve the purpose of transferring the motive power and the torque. One or two joint units 5 are directly fixed at the end of the positioning structure 3 in the first direction, a fixed manner may be integral forming, welding, threaded connection, plug-pin connection or the like, and the joint units 5 and the joint assembly are movably connected in series.

As an alternative implementation of this embodiment, each of the third joining portion 12 of the first adapter structure 1 and the fourth joining portion 22 of the second adapter structure 2 may be formed by the joint unit 5, so as to achieve the purpose of transferring the motive power and the torque. One joint unit 5 is directly fixed at a distal end of the first adapter structure 1 or a proximal end of the second adapter structure, a fixed manner may be integral forming, welding, threaded connection, plug-pin connection or the like, and the joint unit 5 and the joint assembly are movably connected in series.

It needs to be noted that the line-driven motion module may use any of action poses that the line-driven motion module can achieve as an initial action position of driving of the driving mechanism, and the line-driven motion module has advantages of flexible operation and continuous and stable motion.

In the line-driven motion module provided by this embodiment, motion of the control lines 6 is positioned and guided through the positioning structure 3, and the joint assembly and the adapter structures connected to the two ends of the positioning structure 3 bend and transform by using the positioning structure 3 as a reference; when the line-driven motion module is switched between the bent states and the initial state, the first adapter structure 1 and the second adapter structure 2 move correspondingly around the positioning structure 3, an action force and a torque are transferred through connection of the control lines 6, which is conducive to synchronous linkage of the second adapter structure 2 and the first adapter structure 1, and thus the second adapter structure 2 achieves the expected motion action and position; and through whole module structure optimization design, stability of the control lines 6 driving the joint units 5 is improved, motion is continuous, and the tiny run-out error between adjacent joints may be avoided.

### Embodiment 2

This embodiment provides a line-driven motion module, which is different from the line-driven motion module provided by Embodiment 1 in that a limiting channel 32 is not in a shape of a put-through channel, as shown in FIG 17, in this embodiment, four limiting channels 32 are provided and are fitted with and movably connected with four control lines 6, and the four limiting channels 32 are rotationally and symmetrically distributed on a positioning body 31, so as to improve connection stability and achieve the expected transmission actions.

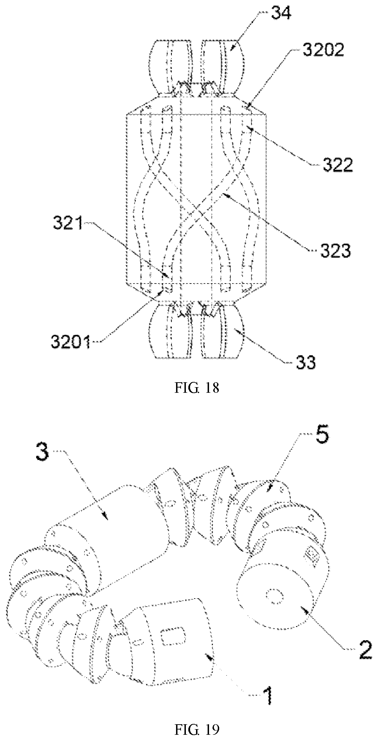

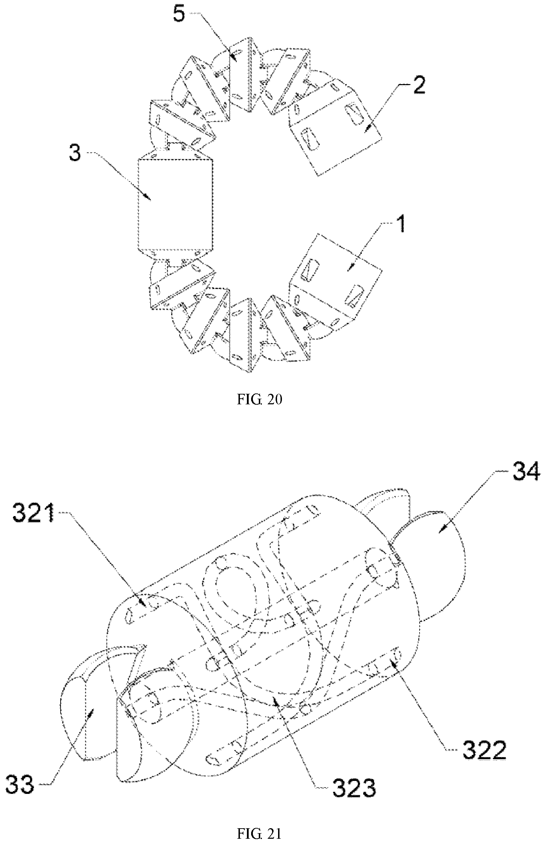

In the line-driven motion module provided by this embodiment, as shown in FIG 18 and FIG 20, each of the limiting channels 32 has a first lead port 3201 at a side close to the first adapter structure 1 and a second lead port 3202 at a side close to the second adapter structure 2, and a first plane containing the first lead port 3201 and an axis of the positioning structure 3 is oriented at an angle of 90 degrees to a second plane containing the second lead port 3202 and the axis of the positioning structure 3; and motion and stress condition of the control lines 6 are changed through the limiting channels 32, so that when drawn by the first adapter structure 1, the control lines 6 automatically act on the second adapter structure 2 and the joint assembly connected between the positioning structure 3 and the second adapter structure 2, the control lines 6 cause the joint assembly and the adapter structures connected to the two ends of the positioning structure 3 to bend and transform by using the positioning structure 3 as a reference, so as to achieve the second bent state in which the second adapter structure 2 and the joint assembly are driven to bend in a linked manner to move in a noncoplanar S shape synchronously with respect to the first adapter structure 1.

Taking one of the limiting channels 32 as an example, as shown in FIG 17 and FIG 18, the limiting channel 32 includes a first straight segment 321, a second straight segment 322 and a curved segment 323; the first straight segment 321 is arranged on a side of the positioning body 31 close to the first adapter body 11, and the second straight segment 322 is arranged on another side of the positioning body 31 close to the second adapter body 21; an extending direction of the first straight segment 321 and an extending direction of the second straight segment 322 are both arranged parallel to an extending direction of the positioning body 31; and the curved segment 323 is arranged between and in communication with the first straight segment 321 and the second straight segment 322, the first lead port 3201 is configured at a proximal end of the first straight segment 321, and the second lead port 3202 is configured at a distal end of the first straight segment 321.

Under a second bent state, for example, when an external force drives the first adapter structure 6 to move in an X-axis extending direction shown in FIG 1, the first adapter structure 6 and the first joint assembly perform the bending motion jointly in the X-axis extending direction, in this case, the second adapter structure 2 and the second joint assembly perform the bending motion jointly in a Y-axis extending direction shown in FIG 1 under a transmission action of the connecting lines 6, a projection of a bending direction of the first joint assembly in the X-Y plane is oriented at an angle of 90 degrees to a projection of the bending direction of the second joint assembly in the X-Y plane, so that the joint assemblies may achieve the expected action poses and positions of a noncoplanar S-shaped curved action process, so as to meet the demanded working condition.

In the line-driven motion module provided by the present application, the first adapter structure 1 and the first joint assembly may bend with respect to the second adapter structure 2 and the second joint assembly, displacement generated by the driving mechanism acting on the first adapter structure 1 in a three-dimensional space with respect to the positioning structure 3 is implemented by compensation through mutual rotation of the adjacent connected joint units 5 between the first adapter structure 1 and the positioning structure 3 and self elastic extending and withdrawing of the control lines 6, so that the first joint assembly performs flexible bending and stretching actions; and in this case, the first end surface 111 and the second end surface 211 remain a position relationship of being arranged originally in parallel in space or being arranged to extend and intersect at an included angle, and the second adapter structure 2 and the second joint assembly remain an original pose without a change. In a flexible motion range of the first joint assembly, the driving mechanism may drive the first adapter structure 1, so that the line-driven motion module performs bending and stretching adjustment on the first joint assembly in a position of a noncoplanar S-shape action process of the line-driven motion module, thus the first joint assembly may achieve the expected motion pose and the position during the bending and stretching action process, so as to meet the demanded working condition. Correspondingly, the driving mechanism may be firstly caused to drive the first adapter structure 1 so as to perform bending and stretching adjustment on the first joint assembly, then the driving mechanism is caused to drive the first adapter structure 1 so as to cause the whole line-driven motion module to generate a noncoplanar S-shape bending action, and thus the demanded working condition is met.

### Embodiment 3

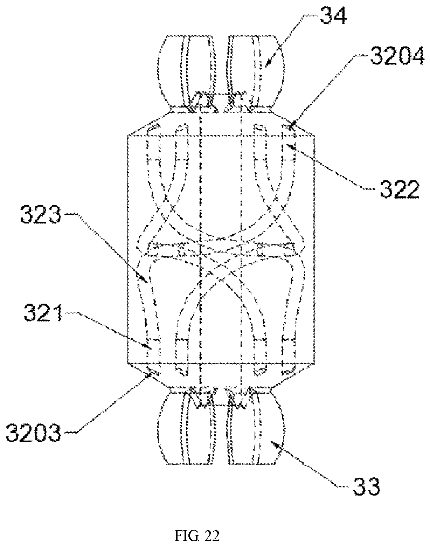

A line-driven motion module provided by this embodiment is different from the line-driven motion module provided by Embodiment 1 in that a limiting channel 32 is not in a shape of a put-through channel, as shown in FIG 21 and FIG 22, in this embodiment, four limiting channels 32 are provided and are fitted with and movably connected with four control lines 6, and the four limiting channels 32 are rotationally and symmetrically distributed on a positioning body 31, so as to improve connection stability and achieve the expected transmission actions.

In the line-driven motion module provided by this embodiment, as shown in FIG 22, each of the limiting channels 32 has a third lead port 3203 at a side close to the first adapter structure 1 and a fourth lead port 3204 at a side close to the second adapter structure 2, and a third plane containing the third lead port 3203 and an axis of the positioning structure 3 is oriented at an angle of 180 degrees to a fourth plane containing the fourth lead port 3204 and the axis of the positioning structure 3; and motion and stress condition of the control lines 6 are changed through the limiting channels 32, so that when drawn by the first adapter structure 1, the control lines 6 automatically act on the second adapter structure 2 and the joint assembly connected between the positioning structure 3 and the second adapter structure 2, the control lines 6 cause the joint assembly and the adapter structures connected to the two ends of the positioning structure 3 to bend and transform by using the positioning structure 3 as a reference, so as to achieve the third bent state in which the second adapter structure 2 and the joint assembly are driven to bend in a linked manner to move in a C shape synchronously with respect to the first adapter structure 1.

Taking one of the limiting channels 32 as an example, as shown in FIG 22, the limiting channel 32 includes a first straight segment 321, a second straight segment 322 and a curved segment 323; an extending direction of the first straight segment 321 and an extending direction of the second straight segment 322 are both arranged parallel to an extending direction of the positioning body 31; and the curved segment 323 is arranged between and in communication with the first straight segment 321 and the second straight segment 322, the third lead port 3203 is configured at a proximal end of the first straight segment 321, and the fourth lead port 3204 is configured at a distal end of the first straight segment 321.

In the third bent state, the first adapter structure 1 and the second adapter structure 2 move towards each other in a circumferential plane along a radial plane of a positioning body 31, so that the joint assembly may achieve the expected action pose and position during the C-shape bending action process, so as to meet the demanded working condition.

In the line-driven motion module provided by the present application, the first adapter structure 1 and the first joint assembly may bend with respect to the second adapter structure 2 and the second joint assembly, displacement generated by the driving mechanism acting on the first adapter structure 1 in a three-dimensional space with respect to the positioning structure 3 is implemented by compensation through mutual rotation of the adjacent connected joint units 5 between the first adapter structure 1 and the positioning structure 3 and self elastic extending and withdrawing of the control lines 6, so that the first joint assembly performs flexible bending and stretching actions; and in this case, the first end surface 111 and the second end surface 211 remain a position relationship of being arranged originally in parallel in space or being arranged to extend and intersect at an included angle, and the second adapter structure 2 and the second joint assembly remain an original pose without a change. In a flexible motion range of the first joint assembly, the driving mechanism may drive the first adapter structure 1, so that the line-driven motion module performs bending and stretching adjustment on the first joint assembly in a position of the C-shape bending action process of the line-driven motion module, thus the first joint assembly may achieve the expected motion pose and the position during the bending and stretching action process, so as to meet the demanded working condition. Correspondingly, the driving mechanism may be firstly caused to drive the first adapter structure 1 so as to perform bending and stretching adjustment on the first joint assembly, then the driving mechanism is caused to drive the first adapter structure 1 so as to cause the whole line-driven motion module to generate a C-shape bending action, and thus the demanded working condition is met.

### Embodiment 4

Minimally invasive surgical forceps include the line-driven motion module in Embodiment 1, Embodiment 2 or Embodiment 3, which therefore has advantages brought by Embodiment 1, Embodiment 2 and Embodiment 3. During a specific use process, when the line-driven motion module self rotates, a support ring 4 may be rotationally connected with a positioning structure 3, and when the line-driven motion module bends in a linked manner, the support ring 4 and the positioning structure 3 may be fixed.

Apparently, the above embodiments are merely examples for clear description, but not for limiting the implementations. Those ordinarily skilled in the art can also make modifications or variations in other different forms based on the above description. All implementations do not need to be and cannot be exhaustively cited here. Apparent modifications or variations derived from this still fall within the protection scope of the present application.

## Claims

1. A line-driven motion module, comprising:
a first adapter structure (1) and a second adapter structure (2) which are spaced apart from each other, wherein the first adapter structure (1) is configured to be connected with an external driving mechanism, and the second adapter structure (2) is configured to be connected with an operating mechanism;
a positioning structure (3), wherein the positioning structure (3) is configured to be connected with an externally-connected support ring (4) and arranged between the first adapter structure (1) and the second adapter structure (2);
a joint assembly, wherein the joint assembly comprises a plurality of interconnected joint units (5), adjacent joint units (5) are configured to be movably connected to each other, and the joint assembly is mechanically coupled and connected between the positioning structure (3) and each of the first and second adapter structures; and
at least two control lines (6), wherein two ends of each control line (6) are fixedly connected with the first adapter structure (1) and the second adapter structure (2), respectively, and the control lines (6) are arranged in all of the joint units (5) and the positioning structure (3) in a penetrating manner to be movably connected with the joint units (5) and the positioning structure (3);
the line-driven motion module has bent states in which the first adapter structure (1) draws the control lines (6) under an external force to enable the second adapter structure (2) and the joint assembly to bend in a linked manner with respect to the first adapter structure (1), and an initial state in which the first adapter structure (1), the positioning structure (3) and the second adapter structure (2) are arranged coaxially; and the line-driven motion module is arranged to switch between the bent states and the initial state.

2. The line-driven motion module according to claim 1, wherein the positioning structure (3) comprises:
a positioning body (31) and at least two limiting channels (32) formed in the positioning body (31), wherein the limiting channels (32) are configured for allowing the control lines (6) to penetrate therethrough.

3. The line-driven motion module according to claim 2, wherein,
an extending direction of each of the limiting channels (32) is arranged parallel to an extending direction of the positioning body (31); and
the line-driven motion module has a first bent state in which the first adapter structure (1) draws the control lines (6) under an external force to enable the second adapter structure (2) and the joint assembly to bend in a linked manner to move in an S shape synchronously with respect to the first adapter structure (1).

4. The line-driven motion module according to claim 3, wherein, in the first bent state, a first end surface (111), remote from a side thereof connected to the joint units (5), of a first adapter body, is arranged parallel to a second end surface (211), remote from a side thereof connected to the joint units (5), of a second adapter body.

5. The line-driven motion module according to claim 2, wherein, each of the limiting channels (32) has a first lead port (3201) at a side close to the first adapter structure (1) and a second lead port (3202) at a side close to the second adapter structure (2), and a first plane containing the first lead port (3201) and an axis of the positioning structure (3) is oriented at an angle of 90 degrees to a second plane containing the second lead port (3202) and the axis of the positioning structure (3); and
the line-driven motion module has a second bent state in which the first adapter structure (1) draws the control lines (6) under an external force to enable the second adapter structure (2) and the joint assembly to bend in a linked manner to move in a noncoplanar S shape synchronously with respect to the first adapter structure (1).

6. The line-driven motion module according to claim 2, wherein each of the limiting channels (32) has a third lead port (3203) at a side close to the first adapter structure (1) and a fourth lead port (3204) at a side close to the second adapter structure (2), and a third plane containing the third lead port (3203) and an axis of the positioning structure (3) is oriented at an angle of 180 degrees to a fourth plane containing the fourth lead port (3204) and the axis of the positioning structure (3); and
the line-driven motion module has a third bent state in which the first adapter structure (1) draws the control lines (6) under an external force to enable the second adapter structure (2) and the joint assembly to bend in a linked manner to move in a C shape synchronously with respect to the first adapter structure (1).

7. The line-driven motion module according to claim 2, wherein,
each limiting channel (32) comprises a first straight segment (321), a second straight segment (322) and a curved segment (323); the first straight segment (321) is arranged on a side of the positioning body (31) close to the first adapter structure (1), and the second straight segment (322) is arranged on another side of the positioning body (31) close to the second adapter structure (2);
an extending direction of the first straight segment (321) and an extending direction of the second straight segment (322) are both arranged parallel to an extending direction of the positioning body (31); and
the curved segment (323) is arranged between and in communication with the first straight segment (321) and the second straight segment (322).

8. The line-driven motion module according to claim 2, wherein,
the positioning structure (3) further comprises a first connecting hole (311) extending in the same direction as an extending direction of the positioning structure (3);
each of the adapter structures has a second connecting hole (112) extending in the same direction as an extending direction of the corresponding adapter structure; and
each of the joint units (5) is provided with a core hole (522), and all the core holes (522), all the second connecting holes (112) and all the first connecting holes (311) together form an intervention channel configured to allow an externally-connected pipe to penetrate therethrough.

9. The line-driven motion module according to any one of claims 1-8, wherein the line-driven motion module further comprises a swiveling state in which the first adapter structure (1) is rotated under an external force to draw the control lines (6) and enable the second adapter structure (2) and the joint assembly to twist synchronously with the first adapter structure (1).

10. The line-driven motion module according to any one of claims 1-8, wherein the positioning structure (3) further comprises:
a first joining portion (33) arranged at an end, close to the first adapter structure (1), of the positioning body (31), wherein, the first joining portion (33) is movably connected to the joint units (5);
a second joining portion (34) arranged at another end, close to the second adapter structure (2), of the positioning body (31), wherein, the second joining portion (34) is configured to be movably connected to the joint units (5); and/or
the first adapter structure (1) comprises a third joining portion (12), wherein, the third joining portion (12) is movably connected to the joint units (5); and
the second adapter structure (2) comprises a fourth joining portion (22), wherein, the fourth joining portion (22) is movably connected to the joint units (5).

11. The line-driven motion module according to any one of claims 1-8, wherein each of the adapter structures further comprises a fitting hole (113) and a fixing portion (114), wherein a corresponding control line (6) is arranged in the fitting hole (113) in a penetrating manner, and the fixing portion (114) adjoins the fitting hole (113), and the fixing portion (114) is fixedly connected to an end of the corresponding control line (6).

12. The line-driven motion module according to any one of claims 1-8, wherein the line-driven motion module comprises at least four control lines (6), and all the control lines (6) are distributed to be rotationally symmetrical with respect to an axis of the positioning structure (3).

13. The line-driven motion module according to any one of claims 1-8, wherein each joint unit (5) comprises:
a rotating member (51), provided with limiting portions (511) in a circumferential direction; and
a limiting base (52) fixedly connected to the rotating member (51), wherein the limiting base (52) is internally provided with at least one receiving cavity (521) for being movably connected to the rotating member (51) of an adjacent joint unit (5), the receiving cavity (521) is further internally provided with limiting members (53), and the limiting members (53) cooperate with the limiting portions (511) of the adjacent joint unit (5) to limit axial rotation of the rotating member (51) of the adjacent joint unit (5).

14. The line-driven motion module according to claim 13, wherein the rotating member (51) is a sphere, and an inner wall surface of the receiving cavity (521) is fitted with an outer wall surface of the rotating member (51) of the adjacent joint unit (5);
two limiting portions (511) are provided, the two limiting portions (511) are spaced apart from each other so as to form a fitting space for limiting the limiting members (53), and the limiting members (53) are movably connected into the fitting space; and
each of the limiting members (53) is a column, wherein, an extending direction of the column is arranged to perpendicularly intersect a plane containing a planar surface of each of the limiting portions (511).

15. The line-driven motion module according to claim 10, wherein each of the joining portions has a structure identical to that of the rotating member (51) or the limiting base (52).

16. Minimally invasive surgical forceps, comprising the line-driven motion module according to any one of claims 1-15.
